# FASCICULE DE BREVET EUROPEEN

(11) **EP 1 412 316 B1**
(45) Date de publication et mention de la délivrance du brevet: **19.10.2011**
(21) Numéro de dépôt: 02767570.1
(22) Date de dépôt: 15.07.2002
(51) Int. Cl.: C07C 51/31, C07C 55/21, C07C 55/14, C07C 51/215, C07C 51/265, C07C 51/235, C07C 51/245, C07C 55/00, C07C 63/00

(54) **PROCEDE D'OXYDATION D'HYDROCARBURES EN ACIDES**
VERFAHREN ZUR OXIDATION VON KOHLENWASSERSTOFFEN IN CARBONSÄUREN
METHOD OF OXIDISING HYDROCARBONS TO ACIDS

(30) Priorité: 03.08.2001 FR 0110427
(43) Date de publication de la demande: 28.04.2004
(73) Titulaire: Rhodia Opérations, 93306 Aubervilliers (FR)
(72) Inventeur: BONNET, Didier, F-69004 Lyon (FR); FACHE, Eric, F-69300 Caluire et Cuire (FR); SIMONATO, Jean-Pierre, F-38360 Sassenage (FR)
(74) Mandataire: Esson, Jean-Pierre
(86) Numéro de dépôt international: PCT/FR2002/002508
(87) Numéro de publication internationale: WO 2003/014055

(56) Documents cités:
- FR-A- 2 806 079
- GB-A- 845 038
- US-A- 4 081 464
- US-A- 6 034 269

## Description

La présente invention concerne un procédé d'oxydation d'hydrocarbures, notamment d'hydrocarbures aliphatiques saturés ramifiés ou non, d'hydrocarbures cycloaliphatiques ou alkylaromatiques et/ou de composés alcools/cétones en composés acides ou polyacides.

Elle se rapporte plus particulièrement à l'oxydation par un agent oxydant contenant de l'oxygène moléculaire, de cyclohexane et/ou du cyclohexanol et/ou cyclohexanone en acide adipique.

L'oxydation du cyclohexane en acide adipique est un procédé qui a été étudié depuis de nombreuses années. En effet, l'acide adipique est un composé chimique important utilisé comme matière première dans de nombreuses fabrications telles que la production de polymères comme les polyamides, polyesters ou polyuréthannes.

Plusieurs procédés de fabrication d'acide adipique à partir d'hydrocarbures tels que benzène, phénol, cyclohexène, cyclohexane ont été proposés.

L'oxydation du cyclohexane soit directement soit en deux étapes est la voie la plus avantageuse pour produire l'acide adipique.

Ainsi, le brevet américain 2,223,493 publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

De nombreux autres brevets et articles décrivent cette réaction d'oxydation directe du cyclohexane en acide adipique. Toutefois, pour obtenir des rendements acceptables de production d'acide adipique, ces documents décrivent l'utilisation de l'acide acétique comme solvant, en présence soit de catalyseur homogène soit de catalyseur hétérogène. On peut citer, à titre d'illustration, l'article paru dans le journal "Chemtech", 555-559 (septembre 1974) dont l'auteur est K. Tanaka qui résume et commente le procédé d'oxydation directe du cyclohexane. On peut également citer les brevets américains 3,231,608 ; 4,032,569 ; 4,158,73 ; 4,263,453 et 5,321,157, le brevet européen 870751 qui décrivent différents systèmes catalytiques homogènes.

Il a également été proposé des procédés d'oxydation directe du cyclohexane en présence de catalyseur hétérogène comme des aluminophosphates substitués par du cobalt, comme dans le brevet européen n°519569.

Le choix du solvant, à savoir l'acide acétique, est une caractéristique importante pour obtenir un taux de transformation du cyclohexane et une production d'acide adipique acceptables. L'utilisation d'un tel solvant présente de nombreux inconvénients provoqués par, par exemple, son caractère corrosif aux conditions de température et de pression utilisées. De plus, l'utilisation de ce solvant pose de nombreux problèmes pour les étapes de séparation et d'extraction de l'acide adipique produit et le recyclage de divers composés.

En effet, en présence d'acide acétique, il est difficile de séparer et extraire du milieu réactionnel les composés sous-produits de l'oxydation tels que la cyclohexanone et le cyclohexanol formés

En outre, l'extraction de l'acide adipique par cristallisation et sa purification sont rendues difficiles car la solubilité à froid de cet acide est plus élevée à 25°C dans l'acide acétique et moins élevée à 80°C dans l'acide acétique que dans l'eau.

La séparation et le recyclage du catalyseur homogène sont également difficiles en présence d'acide acétique. En effet, d'une part un recyclage du catalyseur sans extraction de celui-ci ne permet pas de conserver une activité catalytique suffisante, et d'autre part les opérations de séparation du catalyseur avant recyclage comme décrites notamment dans les brevets français n° 2722783, 2746671, sont complexes et coûteuses.

De plus, ce solvant impose de réaliser une déshydratation difficile et coûteuse du milieu réactionnel. GB 845038 décrit un procédé d'oxydation des xylènes dans l'acide benzoique.

Il a également été proposé quelques procédés d'oxydation en une seule étape du cyclohexane en acide adipique sans utilisation d'acide acétique. Certains proposent de réaliser cette réaction en l'absence de solvants, d'autres avec des solvants tels que des esters organiques comme les acétates (US 4,098,817), de l'acétone (US 2,589,648) ou encore des alcools comme le butanol, le méthanol, le cyclohexanol ou l'acétonitrile (Ep 784045).

Ces procédés conduisent généralement à des sélectivités en acide adipique très faibles. Par ailleurs, les solvants utilisés présentent souvent une faible stabilité dans les conditions d'oxydation de l'hydrocarbure comme le cyclohexane. Cette faible stabilité provoque une consommation importante du solvant ce qui rend inexploitable de tels procédés.

Un des buts,de la présente invention est de proposer un procédé d'oxydation d'hydrocarbures en une seule étape pour produire des acides ou polyacides, dans un milieu liquide aux conditions de la réaction d'oxydation et permettant une séparation de l'acide produit et un recyclage notamment du catalyseur par des opérations simples.

A cet effet, l'invention propose un procédé d'oxydation d'hydrocarbures aliphatiques ou cycloaliphatiques saturés substitués ou non ou d'hydrocarbures alkylaromatiques en acides ou polyacides dans un milieu liquide par un agent d'oxydation comprenant de l'oxygène moléculaire, caractérisé en ce qu'un des constituants du milieu liquide est un composé organique acide de formule générale (I) suivante : dans laquelle : 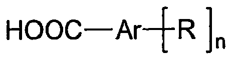
- Ar représente un radical aromatique comprenant un cycle aromatique ou plusieurs cycles aromatiques sous forme condensée,
- n représente un nombre entier pouvant être égal à 1, 2 ou 3,
   R représente un radical de formule générale (II) suivante : 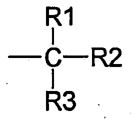
dans laquelle :
R1, R2, R3 identiques ou différents représentent une chaîne alkyle comprenant de 1 à 4 atomes de carbone ou un atome de fluor, de chlore ou de brome.

Selon l'invention la réaction d'oxydation est réalisée en présence d'un catalyseur à base de manganèse.

Selon l'invention, le composé de formule générale (I) est avantageusement choisi dans la liste des acides benzoiques, naphtaléniques avantageusement substitués par des groupements tertioalkyles tels que le tertiobutyle ou par des radicaux trifluorocarbonés. On peut citer comme acides convenables pour l'invention l'acide 3,5-ditertiobutylbenzoique, l'acide 3,5-ditrifluorométhylbenzoïque, l'acide 4-trifluorométhylbenzoïque, l'acide 4-tertiobutylbenzoïque.

Ces composés acides pourront être au moins partiellement miscibles avec le ou les hydrocarbures à oxyder, aux conditions de température et de pression mises en oeuvre pour réaliser la réaction d'oxydation, et avantageusement peu solubles dans l'eau c'est à dire une solubilité inférieure à 10 % en poids à température ambiante(10°C - 30°C)..

par au moins partiellement miscible, on entend qu'aux conditions de la réaction d'oxydation, la solubilité d'un composé dans l'autre soit au moins supérieure à 2% en poids, et qu'au moins une phase liquide homogène comprenant au moins une partie des hydrocarbures à oxyder et du composé acide soit formée.

Dans un mode de réalisation préféré de l'invention, la miscibilité entre l'hydrocarbure et le composé acide ci-dessus est telle qu'aux conditions de mise en oeuvre de l'invention, ces deux composés forment une seule phase liquide homogène.

Toutefois, il est possible sans sortir du cadre de l'invention, d'utiliser des composés organiques présentant une solubilité dans l'eau supérieure à celle indiquée précédemment si le coefficient de partage de ce composé entre la ou les phases organiques du milieu réactionnel constituées essentiellement par l'hydrocarbure à oxyder, les intermédiaires d'oxydation et la phase non organique comprenant l'eau formée pendant la réaction d'oxydation permet d'obtenir une concentration du composé organique de formule générale (I) dans ladite phase aqueuse inférieure à 10 % en poids.

Selon une autre caractéristique de l'invention, la concentration en composé acide dans le milieu réactionnel est déterminée pour obtenir un rapport molaire entre le nombre de mole d'acide et le nombre de mole de métal manganèse formant le catalyseur compris entre 0,5 et 100 000, de préférence entre 1 et 5000.

La concentration en composé acide dans le milieu liquide d'oxydation peut varier dans de larges limites. Ainsi, elle peut être comprise entre 1 et 99 % en poids par rapport au poids total du milieu liquide, plus avantageusement elle peut être comprise entre 2 et 50 % en poids du milieu liquid.

Il est également possible, sans pour cela sortir du cadre de l'invention, d'utiliser le composé acide en association avec un autre composé qui peut notamment avoir comme effet d'améliorer la productivité et/ou la sélectivité de la réaction d'oxydation en acide adipique, et notamment la solubilisation de l'oxygène.

Comme exemples de tels composés, on peut citer, en particulier, les nitriles, les composés hydroxyimides les composés halogénés, plus avantageusement les composés fluorés. Comme composés plus particulièrement convenables, on peut citer les nitriles comme l'acétonitrile, le benzonitrile, les imides appartenant à la famille décrite dans la demande de brevet Ep 0824962, et plus particulièrement la N-hydroxysuccinimide (NHS) ou la N-hydroxyphtalimide (NHPI), des dérivés halogénés comme le dichlorométhane, les composés fluorés comme :
Hydrocarbures aliphatiques fluorés ou perfluorés cycliques ou acycliques,
hydrocarbures fluorés aromatiques tels le perfluorotoluène,
perfluorométhylcyclohexane, perfluorohexane, perfluoroheptane,
perfluorooctane, perfluorononane, perfluorodécaline, perfluorométhyldécaline,
α,α,α-trifluorotoluène, 1,3-bis(méthyl trifluoro)benzène.
Esters perfluorés ou fluorés tels que perfluorooctanoates d'alkyle,
perfluoronanoates d'alkyle
Cétones fluorées ou perfluorées telles que acétone perfluorée
Alcools fluorés ou perfluorés tels que hexanol, octanol, nonanol, décanol perfluorés, t-butanol perfluoré, isopropanol perfluoré, hexafluoro-1,1,1,3,3,3-propanol-2
Nitriles fluorés ou perfluorés tels que acétonitrile perfluoré
Acides fluorés ou perfluorés tels que acides trifluorométhyl benzoïque, acide pentafluorobenzoique, acide hexanoique, heptanoique, octanoique, nonanoique perfluorés, acide adipique perfluoré
Halogénures fluorés ou perfluorés tels que iodo octane perfluoré, bromooctane perfluoré
Amines fluorées ou perfluorées tels que tripropylamine perfluorée, tributylamine perfluorée, tripentylamine perfluorée :

L'oxydation est réalisée en présence d'un catalyseur. Ce catalyseur comprend comme élément métallique catalytiquement actif du manganèse.

Ce catalyseur est mis en oeuvre soit sous forme de composés avantageusement au moins partiellement solubles dans le milieu liquide d'oxydation aux conditions de mise en oeuvre de la réaction d'oxydation (catalyse homogène), soit supportés, absorbés ou liés à un support inerte tel que silice, alumine, par exemple (catalyse hétérogène).

Le catalyseur est de préférence, notamment aux conditions de mise en oeuvre de la réaction d'oxydation :
Soit soluble dans l'hydrocarbure à oxyder,
Soit soluble dans le composé acide,
Soit soluble dans le mélange hydrocarbure/composé acide formant une phase liquide homogène aux conditions de mise en oeuvre de la réaction.

Selon un mode de réalisation préféré de l'invention, le catalyseur utilisé est soluble dans l'un de ces milieux à température ambiante ou à la température de recyclage de ces milieux dans une nouvelle oxydation.

Par le terme soluble, on entend que le catalyseur soit au moins partiellement soluble dans le milieu considéré.

Dans le cas d'une catalyse hétérogène, les éléments métalliques catalytiquement actifs sont supportés ou incorporés dans une matrice minérale micro ou mésoporeuse ou dans une matrice polymérique ou sont sous forme de complexes organométalliques greffés sur un support organique ou minéral. Par incorporé, on entend que le métal est un élément du support ou que l'on travaille avec des complexes stériquement piégés dans des structures poreuses dans les conditions de l'oxydation.

Dans un mode de réalisation préféré de l'invention, le catalyseur homogène ou hétérogène est constitué de sels ou de complexes de manganèse. La concentration pondérale en métal exprimé en poids de manganèse dans le milieu liquide d'oxydation est avantageusement supérieure à 10 ppm, de préférence comprise entre 50 ppm et 25 000 ppm, et encore plus avantageusement entre 50 ppm et 5 000 ppm.

L'invention s'applique plus particulièrement à l'oxydation de composés cycloaliphatiques tels que le cyclohexane, le cyclododécane en diacides linéaires correspondants, l'acide adipique, l'acide dodécanoïque.

Selon un mode de réalisation préféré de l'invention, elle concerne l'oxydation directe du cyclohexane en acide adipique, par un gaz contenant de l'oxygène, en milieu liquide et en présence d'un catalyseur au manganèse.

La réaction d'oxydation est mise en oeuvre à une température comprise entre 50°C et 200°C, de préférence entre 70°C et 180°C. Elle peut être réalisée sous pression atmosphérique. Toutefois, elle est généralement mise en oeuvre sous pression pour maintenir les composants du milieu réactionnel sous forme liquide. La pression peut être comprise entre 10 KPa (0,1 bar) et 20000 KPa (200 bars), de préférence entre 100Kpa (1 bar) et 10000 Kpa (100 bar).

L'oxygène utilisé peut être sous forme pure ou en mélange avec un gaz inerte tel que l'azote ou l'hélium. On peut également utiliser de l'air plus ou moins enrichi en oxygène. La quantité d'oxygène alimentée dans le milieu est avantageusement comprise entre 1 et 1000 moles par mole de composés à oxyder.

Le procédé d'oxydation peut être réalisé de manière continue ou selon un procédé discontinu. Avantageusement, le milieu réactionnel liquide sorti du réacteur est traité selon des procédés connus permettant d'une part de séparer et récupérer le diacide produit et d'autre part recycler les composés organiques non oxydés ou partiellement oxydés comme le cyclohexane, le cyclohexanol et/ou la cyclohexanone, le catalyseur et le composé acide.

Le catalyseur, outre le manganèse, peut également comporter d'autres composés à base de métaux choisis dans le groupe comprenant le cobalt, le cuivre, le cérium, le brome, le vanadium, le chrome, le zirconium, l'hafnium ou une association de certains de ces éléments . Il est notamment interressant d'associer au manganèse un élément comme le cobalt.

Il est avantageux de mettre en oeuvre également un composé initiateur de la réaction d'oxydation, tel que par exemple une cétone, un aldéhyde ou un hydroperoxyde. La cyclohexanone et l'hydroperoxyde de cyclohexyle qui sont des intermédiaires réactionnels dans le cas de l'oxydation du cyclohexane, sont tout particulièrement indiqués. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation. Il peut être introduit dès le début de la réaction.

L'oxydation peut également être mise en oeuvre en présence d'eau introduite dès le stade initial du procédé.

Comme indiqué ci-dessus, le mélange réactionnel issu de l'oxydation est soumis à différentes opérations de séparation de certains de ses constituants pour, par exemple, permettre leur recyclage au niveau de l'oxydation et la récupération des acides produits.

Selon une première variante du procédé, on peut soumettre tout d'abord le mélange réactionnel brut à un refroidissement à une température de 16°C à 30°C par exemple, ce qui occasionne la cristallisation d'au moins une partie de l'acide formé. On obtient ainsi un milieu comprenant une phase solide constituée essentiellement d'acide, au moins une phase liquide organique contenant essentiellement le composé à oxyder n'ayant pas réagi, éventuellement le composé acide et les intermédiaires d'oxydation, (ou plusieurs phases organiques si le composé acide et l'hydrocarbure ne sont pas totalement miscibles à basse température) et une phase liquide aqueuse contenant essentiellement des sous produits acides de l'oxydation et l'eau formée. Le catalyseur peut se trouver dans une des phases organiques s'il est soluble dans ladite phase, ou dans la phase aqueuse inférieure.

Après filtration ou centrifugation du solide, on procède s'il y a lieu à la séparation par décantation des phases liquides organiques et aqueuse constituant le filtrat ou le centrifugat : la ou les phases organiques peuvent être recyclées dans une nouvelle réaction d'oxydation.

Il peut être avantageux de procéder, préalablement à l'opération de cristallisation de l'acide, à une concentration du mélange réactionnel.

Selon une deuxième variante du procédé, on peut soutirer à chaud le mélange réactionnel brut final, par exemple à une température pouvant atteindre 75°C. Le mélange réactionnel décante alors en au moins deux phases liquides : une ou plusieurs phases organiques contenant essentiellement l'hydrocarbure n'ayant pas réagi, le composé acide, les intermédiaires d'oxydation et une phase liquide aqueuse contenant essentiellement les acides formés et l'eau formée. Selon la solubilité et la nature du catalyseur celui-ci peut être présent dans la ou les phases organiques, récupéré par séparation solide/liquide avant précipitation ou cristallisation de l'acide formé dans le cas d'une catalyse hétérogène ou s'il est soluble dans la phase aqueuse, extrait par extraction liquide/liquide, sur résine ou électrodialyse.

Comme dans la première variante, on procède à la séparation par décantation des phases liquides : la ou les phases organiques peuvent être recyclées dans une nouvelle réaction d'oxydation.

Dans ces modes de réalisation, le composé acide utilisé conformément à l'invention est généralement contenu ou forme un élément essentiel de la ou des phases organiques. En conséquence, après séparation de l'acide formé et éventuellement de la phase liquide contenant l'eau formée, les sous-produits d'oxydation et le catalyseur, le composé acide sont recyclés dans l'étape d'oxydation avec l'hydrocarbure n'ayant pas été oxydé et les intermédiaires d'oxydation.

Par ailleurs, si le composé acide est solide dans une phase de traitement du milieu réactionnel, il sera avantageusement séparé et récupéré par mise en oeuvre des procédés de séparation solide/liquide soit avant traitement du milieu réactionnel pour récupérer l'acide produit, soit avec l'acide produit. Dans ce dernier cas, l'acide produit pourra être récupéré par extraction à l'eau.

Il est remarquable que la récupération du composé acide soit presque totale, c'est à dire qu'une quantité très faible, pratiquement non mesurable de composé acide soit transformée pendant la réaction ou entraînée avec l'acide adipique séparé. Ainsi, le taux de récupération du composé acide est supérieure à 97 %.

Dans ces exemples de modes de réalisation de l'invention, de l'eau peut être ajoutée au milieu réactionnel pour obtenir une meilleure dissolution des sous produits acides de l'oxydation et une meilleure récupération de l'acide formé.

La récupération de l'acide est généralement réalisée par précipitation lors du refroidissement du milieu réactionnel. L'acide ainsi récupéré peut être purifié selon des techniques habituelles et décrites dans de nombreux brevets. On peut citer, à titre d'exemple, les brevets français n° 2 749 299 et 2 749 300.

Si la phase liquide non organique ou aqueuse contient le catalyseur celui-ci est extrait soit avant la cristallisation de l'acide formé par précipitation ou extraction selon des procédés connus comme l'extraction liquide - liquide, l'électrodialyse, traitement sur résines échangeuses d'ions par exemple, soit après cristallisation de l'acide formé par des techniques d'extraction décrites ci-dessus ou analogues.

D'autres avantages, détails de l'invention apparaîtront plus clairement au vu des exemples donnés ci-dessous uniquement à titre indicatif et d'illustration.

### Exemple 1

Dans un autoclave de 125 mL en titane muni de moyens de chauffage par collier chauffant, d'une turbine et de moyens d'introduction de gaz et de régulation de pression on charge :
- 0,0344 g de Mn(acac)₃ (107 ppm exprimés en Mn) (Acac : acétylacétonate)
- 0,5594 g (5,71 mmol) de cyclohexanone
- 45,0965g (536,9 mmol) de cyclohexane
- 5,0157 g (28,17 mmol) d'acide 4-tertiobutylbenzoïque

Après fermeture du réacteur, une agitation à 1000 tours par minute est appliquée, 125 bar d'air (à 20°C) sont introduits et le chauffage est mis en route. La température atteint 140°C dans la masse en 10 minutes et est maintenue durant 25 minutes.

Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Le taux de transformation du cyclohexane (TT) est de 7,17%.

Les résultats des analyses sont rassemblés dans le tableau I ci-dessous.

**Tableau I**

| Produits | ST % |
|---|---|
| Acide adipique | 53,6 |
| Acide glutarique | 11,8 |
| Acide succinique | 3,5 |

L'acide tertiobutylbenzoïque est quantitativement dosé en fin d'essai.

ST % = sélectivité en composé indiqué à la première colonne par rapport au cyclohexane transformé.

TT % = taux de transformation du cyclohexane

### Exemples 2 à 6

Dans un autoclave de 30 mL en alloy C22 muni de moyens de chauffage et agité par secousses on charge :
- 0,0033 g de Mn(acac)₃ (106 ppm exprimés en Mn) (Acac : acétylacétonate)
- 0,0585 g (0,597 mmol) de cyclohexanone
- 5,00 g (58,9 mmol) de cyclohexane
- 2% molaire de composé acide par rapport au cyclohexane

Après fermeture du réacteur et mise sous agitation, 100 bar d'air (à 20°C) sont introduits et le chauffage est mis en route. Le réacteur est ainsi agité 3 heures à 120°C. Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Le taux de transformation du cyclohexane (TT) basé sur le dosage des produits en fin d'essai est présenté dans le tableau II, pour chaque composé acide utilisé.

**Tableau II**

| Ex | Composé acide | TT Cyclohexane % |
|---|---|---|
| 2 (comparatif) | sans | 0,48 |
| 3 | Acide 4-trifluoromethylbenzoïque | 2,32 |
| 4 | Acide 4 -tertiobutylbenzoïque | 3,64 |
| 5 (comparatif) | Acide naphtalénique | 0,94 |
| 6 (comparatif) | Acide benzoïque | 1,65 |

### Exemples 7 à 10

Dans un autoclave de 30 mL en alloy C22 muni de moyens de chauffage et agité par secousses on charge :
- x g de Mn(acac)₃ pour obtenir une concentration de catalyseur exprimée en ppm de Mn indiquée dans le tableau III ci-dessous
- 0,0525 g de cyclohexanone
- 4,5 g de cyclohexane
- 0,500g d'acide 4-tertiobutylbenzoïque

Après fermeture du réacteur et mise sous agitation, 100 bar d'air (à 20°C) sont introduits et le chauffage est mis en route. Le réacteur est ainsi agité 3 heures à 120°C. Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Les TT de cyclohexane et ST en acide adipique sont présentés dans le tableau III pour différentes concentrations en catalyseur.

**Tableau III**

| EX | 7 | 8 | 9 | 10 |
|---|---|---|---|---|
| Mn (ppm) | 10 | 100 | 1000 | 10000 |
| ST AdOH % | 29,7 | 39,7 | 38 | 26,0 |
| TT cyclo % | 3,86 | 4,27 | 3,03 | 1,79 |

| | | | | |
|---|---|---|---|---|
| ppm : partie par million massique de manganèse métal dans le milieu réactionnnel | | | | |

### Exemple 11

Dans un autoclave de 180 mL en titane muni de moyens de chauffage, on charge :
- 0,0154 g de Mn(acac)₃ (Acac : acétylacétonate)
- 0,0066 g de Co(acac)₃
- 0,5089 g de cyclohexanone
- 45,085 g de cyclohexane
- 5,002 g d'acide 4-tertiobutylbenzoïque

Après fermeture du réacteur, une agitation à 1000 tours par minute est appliquée, 106 bar d'air (à 20°C) sont introduits et le chauffage est mis en route. La température atteint 130°C dans la masse en 10 minutes et est maintenue durant 30 minutes.

Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Le taux de transformation du cyclohexane (TT) est de 7.8 %.

La quantité de produits dosés en fin d'essai est indiquée ci dessous
Acide adipique : 2.61 g
Acide glutarique : 0.48 g
Acide succinique : 0.13 g
Cyclohexanol : 1.37 g
Cyclohexanone :0.61 g

### Exemple 12

L'essai 11 est répété avec la charge suivante :
- 0,0158 g de Mn(acac)₃ (Acac : acétylacétonate)
- 0,0060 g de Co(acac)₃
- 0,5164 g de cyclohexanone
- 45,109 g de cyclohexane
- 5,07 g d'acide 4-tertiobutylbenzoïque

Après fermeture du réacteur, une agitation à 1000 tours par minute est appliquée, 75 bar d'air (à 20°C) sont introduits et le chauffage est mis en route. La pression du réacteur est maintenue à 100 bar avec une pression partielle en oxygène de 20 bar maintenue à l'aide d'une réserve d'oxygène pur. La température est maintenue à 130°C dans la masse durant 40 minutes.

Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Le taux de transformation du cyclohexane (TT) est de 10.1 %.

La quantité de produits dosés en fin d'essai est indiquée ci dessous :
Acide adipique : 3.94 g
Acide glutarique : 0.71 g
Acide succinique : 0.201 g
Cyclohexanol : 1.4 g
Cyclohexanone : 0.51 g

### Exemple 13

L'essai 11 est répété avec la charge suivante :
- 0,0144 g de MnBr₂, 4H₂O
- 0,0095 g de Co(acac)₃
- 0,5050 g de cyclohexanone
- 45,018 g de cyclohexane
- 5,037 g d'acide 4-tertiobutylbenzoïque

Après fermeture du réacteur, une agitation à 1000 tours par minute est appliquée, 75 bar d'air (à 20°C) sont introduits et le chauffage est mis en route. La pression du réacteur est maintenue à 100 bar avec une pression partielle en oxygène de 20 bar maintenue à l'aide d'une réserve d'oxygène pur. La température est maintenue à 130°C dans la masse durant 30 minutes.

Après refroidissement et dépressurisation, le réacteur est ouvert. Le mélange réactionnel comprend une phase comprenant le cyclohexane et un précipité. L'ensemble est dissous dans de l'acide acétique et les produits organiques sont dosés par chromatographie en phase gazeuse avec étalonnage interne.

Le taux de transformation du cyclohexane (TT) est de 7.8 %.

La quantité de produits dosés en fin d'essai est indiquée ci dessous
Acide adipique : 2.71g
Acide glutarique : 0.48 g
Acide succinique : 0.13 g
Cyclohexanol : 1.38 g
Cyclohexanone :0.74 g

## Revendications

1. Procédé d'oxydation d'hydrocarbures aliphatiques ou cycloaliphatiques saturés substitués ou non ou d'hydrocarbures alkylaromatiques et/ou d'alcools et de cétones en milieu liquide par un agent d'oxydation comprenant de l'oxygène moléculaire en acides ou polyacides, **caractérisé en ce que** la réaction est réalisée en présence d'un catalyseur à base de manganèse et d'un composé organique acide de formule générale (I) suivante : 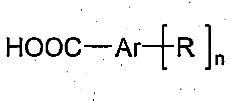 dans laquelle :
- Ar représente un radical aromatique comprenant un cycle aromatique ou plusieurs cycles aromatiques sous forme condensée,
- n représente un nombre entier pouvant être égal à 1, 2 ou 3, R représente un radical de formule générale (II) suivante : 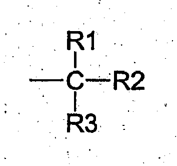
dans laquelle :
R1, R2, R3 identiques ou différents représentent une chaîne alkyle comprenant de 1 à 4 atomes de carbone ou un atome de fluor, de chlore ou de brome.

2. Procédé selon la revendication 1, **caractérisé en ce que** l'hydrocarbure à oxyder est au moins partiellement miscible avec le composé acide aux conditions de mise en oeuvre de la réaction d'oxydation.

3. Procédé selon l'une des revendications 1 à 2, **caractérisé en ce que** le composé acide est choisi dans le groupe comprenant les acides benzoiques, naphtaléniques.

4. Procédé selon la revendication 3, **caractérisé en ce que** les acides benzoiques, naphtaléniques sont substitués par des groupements tertioalkyles, fluorocarbonés.

5. Procédé selon la revendication 3 ou 4, **caractérisé en ce que** le composé acide est choisi dans le groupe comprenant l'acide 3,5-ditertiobutylbenzoique, l'acide 3,5-ditrifluorométhylbenzoïque, l'acide 4-trifluorométhylbenzoïque, l'acide 4-tertiobutylbenzoïque

6. Procédé selon l'une des revendications précédentes, **caractérisé en ce que** le pourcentage pondéral de composé acide dans le milieu liquide est compris entre 1 et 99 % en poids par rapport au poids total du milieu liquide.

7. Procédé selon la revendication 6, **caractérisé en ce que** le pourcentage pondéral précité est compris entre 2 et 50 % en poids.

8. Procédé selon l'une des revendications précédentes, **caractérisé en ce que** le catalyseur est soluble dans le milieu liquide aux conditions de mise en oeuvre de la réaction d'oxydation.

9. Procédé selon l'une des revendications 1 à 7, **caractérisé en ce que** le catalyseur est insoluble dans le milieu liquide aux conditions de mise en oeuvre de la réaction d'oxydation.

10. Procédé selon la revendication 9, **caractérisé en ce que** le catalyseur est un catalyseur supporté comprenant un support minéral ou polymérique.

11. Procédé selon l'une des revendications précédentes, **caractérisé en ce que** l'oxydation est réalisée en présence d'un composé choisi dans la famille comprenant les nitriles, les composés hydroxyimides, les composés halogénés.

12. Procédé selon la revendication 11, **caractérisé en ce que** le composé précité est un composé nitrile choisi dans le groupe comprenant l'acétonitrile, le benzonitrile.

13. Procédé selon la revendication 11, **caractérisé en ce que** le composé précité est un composé hydroxyimide choisi dans le groupe comprenant la N-hydroxysuccinimide et la N-hydroxyphtalimide.

14. Procédé selon la revendication 11, **caractérisé en ce que** le composé précité est un composé halogéné choisi dans le groupe comprenant les hydrocarbures aliphatiques fluorés ou perfluorés cycliques ou acycliques, les hydrocarbures fluorés aromatiques tels le perfluorotoluène, perfluorométhylcyclohexane, perfluorohexane, perfluoroheptane, perfluorooctane, perfluorononane, perfluorodécaline, perfluorométhyldécaline, α,α,α-trifluorotoluène, 1,3-bis(méthyl trifluoro)benzène, les esters perfluorés ou fluorés tels que perfluorooctanoates d'alkyle, perfluoronanoates d'alkyle, les cétones fluorées ou perfluorées telles que acétone perfluorée, les alcools fluorés ou perfluorés tels que hexanol, octanol, nonanol, décanol perfluorés, t-butanol perfluoré, isopropanol perfluoré, hexafluoro-1,1,1,3,3,3-propanol-2, les nitriles fluorés ou perfluorés tels que acétonitrile perfluoré, les acides fluorés ou perfluorés tels que acides trifluorométhyl benzoïque, acide pentafluorobenzoique, acide hexanoique, heptanoique, octanoique, nonanoique perfluorés, acide adipique perfluoré, les halogénures fluorés ou perfluorés tels que iodo octane perfluoré, bromooctane perfluoré, les amines fluorées ou perfluorées tels que tripropylamine perfluorée, tributylamine perfluorée, tripentylamine perfluorée

15. Procédé selon l'une des revendications précédentes, **caractérisé en ce que** l'hydrocarbure à oxyder est choisi dans le groupe comprenant le cyclohexane, le cyclododecane.

16. Procédé selon la revendication 15, **caractérisé en ce que** l'acide produit est l'acide adipique ou l'acide dodécanedioïque.

17. Procédé selon l'une des revendications précédentes, **caractérisé en ce que** le milieu liquide, après oxydation, est décanté, en au moins une phase organique formée par l'hydrocarbure non oxydé, le composé acide, lesdites phases organiques étant recyclées dans une nouvelle oxydation, et au moins une phase aqueuse contenant l'acide produit.

18. Procédé selon la revendication 17, **caractérisé en ce que** l'acide est extrait de la phase aqueuse par cristallisation.

19. Procédé selon l'une des revendications 9 et 17 ou 18, **caractérisé en ce que** le catalyseur est recyclé avec la ou les phases organiques.

20. Procédé selon l'une des revendications 10 et 17 ou 18, **caractérisé en ce que** le catalyseur est séparé du milieu liquide par décantation ou séparation solide/liquide.

21. Procédé selon l'une des revendications 17 ou 18, **caractérisé en ce que** le catalyseur soluble dans la phase aqueuse est extrait par extraction liquide/liquide, séparation sur résines, ou par électrodialyse.

22. Procédé selon l'une des revendications précédentes, **caractérisé en ce que** le catalyseur comprend des éléments dopants choisis dans le groupe comprenant le cobalt, le cuivre, le cérium, le vanadium, le chrome, le brome, le zirconium, l'hafnium ou une association de certains de ces éléments.

23. Procédé selon l'une des revendications précédentes, **caractérisé en ce que** le catalyseur comprend du manganèse et du cobalt.

24. Procédé selon l'une des revendications précédentes, **caractérisé en ce que** la concentration en catalyseur exprimé en manganèse dans le milieu d'oxydation est supérieure à 10 ppm,.

25. Procédé selon l'une des revendications précédentes, **caractérisé en ce que** la concentration en catalyseur exprimée en manganèse dans le milieu d'oxydation est comprise entre 10 ppm et 5000 ppm.

## Claims

1. Process for the oxidation of substituted or unsubstituted saturated aliphatic or cycloaliphatic hydrocarbons or of alkylaromatic hydrocarbons and/or of alcohols and ketones to acids or polyacids in a liquid medium by an oxidizing agent comprising molecular oxygen, **characterized in that** the reaction is carried out in the presence of a manganese-based catalyst and an organic acid compound of the following general formula (I): 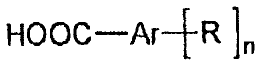 in which:
- Ar represents an aromatic radical containing an aromatic ring or several aromatic rings in condensed form,
- n represents an integer which may be equal to 1, 2 or 3,
R represents a radical of the following general formula (II): 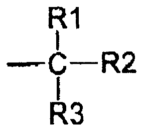 in which:
R1, R2 and R3, which are identical or different, represent an alkyl chain containing from 1 to 4 carbon atoms or a fluorine, chlorine or bromine atom.

2. Process according to Claim 1, **characterized in that** the hydrocarbon to be oxidized is at least partially miscible with the acid compound under the conditions of implementation of the oxidation reaction.

3. Process according to either of Claims 1 and 2, **characterized in that** the acid compound is chosen from the group comprising benzoic and naphthalenic acids.

4. Process according to Claim 3, **characterized in that** the benzoic and naphthalenic acids are substituted by tert-alkyl or fluorocarbon-based groups.

5. Process according to Claim 3 or 4, **characterized in that** the acid compound is chosen from the group comprising 3,5-di-tert-butylbenzoic acid, 3,5-ditrifluoromethylbenzoic acid, 4-trifluoromethylbenzoic acid and 4-tert-butylbenzoic acid.

6. Process according to one of the preceding claims, **characterized in that** the percentage by weight of acid compound in the liquid medium is between 1 and 99% by weight relative to the total weight of the liquid medium.

7. Process according to Claim 6, **characterized in that** the abovementioned percentage by weight is between 2 and 50% by weight.

8. Process according to one of the preceding claims, **characterized in that** the catalyst is soluble in the liquid medium under the conditions of implementation of the oxidation reaction.

9. Process according to one of Claims 1 to 7, **characterized in that** the catalyst is insoluble in the liquid medium under the conditions of implementation of the oxidation reaction.

10. Process according to Claim 9, **characterized in that** the catalyst is a supported catalyst comprising an inorganic or polymeric support.

11. Process according to one of the preceding claims, **characterized in that** the oxidation is carried out in the presence of a compound chosen from the family comprising nitriles, hydroxyimide compounds and halogenated compounds.

12. Process according to Claim 11, **characterized in that** the abovementioned compound is a nitrile compound chosen from the group comprising acetonitrile and benzonitrile.

13. Process according to Claim 11, **characterized in that** the abovementioned compound is a hydroxyimide compound chosen from the group comprising N-hydroxysuccinimide and N-hydroxyphthalimide.

14. Process according to Claim 11, **characterized in that** the abovementioned compound is a halogenated compound chosen from the group comprising cyclic or acyclic fluorinated or perfluorinated aliphatic hydrocarbons or fluorinated aromatic hydrocarbons, such as perfluorotoluene, perfluoromethylcyclohexane, perfluorohexane, perfluoroheptane, perfluorooctane, perfluorononane, perfluorodecalin, perfluoromethyldecalin, α,α,α-trifluorotoluene or 1,3-bis(trifluoromethyl)benzene, perfluorinated or fluorinated esters, such as perfluoro(alkyl octanoate)s or perfluoro(alkyl nonanoate)s, fluorinated or perfluorinated ketones, such as perfluoroacetone, fluorinated or perfluorinated alcohols, such as perfluorohexanol, perfluorooctanol, perfluorononanol, perfluorodecanol, perfluoro-t-butanol, perfluoroisopropanol or 1,1,1,3,3,3-hexafluoro-2-propanol, fluorinated or perfluorinated nitriles, such as perfluoroacetonitrile, fluorinated or perfluorinated acids, such as trifluoromethylbenzoic acids, pentafluorobenzoic acid, perfluorohexanoic acid, perfluoroheptanoic acid, perfluorooctanoic acid, perfluorononanoic acid or perfluoroadipic acid, fluorinated or perfluorinated halides, such as perfluoroiodooctane or perfluorobromo-octane, fluorinated or perfluorinated amines, such as perfluorotripropylamine, perfluorotributylamine or perfluorotripentylamine.

15. Process according to one of the preceding claims, **characterized in that** the hydrocarbon to be oxidized is chosen from the group comprising cyclohexane and cyclododecane.

16. Process according to Claim 15, **characterized in that** the acid produced is adipic acid or dodecanedioic acid.

17. Process according to one of the preceding claims, **characterized in that** the liquid medium, after oxidation, is separated by settling into at least one organic phase formed by the nonoxidized hydrocarbon and the acid compound, the said organic phases being recycled in a further oxidation, and at least one aqueous phase containing the acid produced.

18. Process according to Claim 17, **characterized in that** the acid is extracted from the aqueous phase by crystallization.

19. Process according to either of Claims 9 and 17 or 18, **characterized in that** the catalyst is recycled with the organic phase(s).

20. Process according to either of Claims 10 and 17 or 18, **characterized in that** the catalyst is separated from the liquid medium by separation by settling or solid/liquid separation.

21. Process according to either of Claims 17 and 18, **characterized in that** the catalyst which is soluble in the aqueous phase is extracted by liquid/liquid extraction, separation over resins or by electrodialysis.

22. Process according to one of the preceding claims, **characterized in that** the catalyst comprises doping elements chosen from the group comprising cobalt, copper, cerium, vanadium, chromium, bromine, zirconium, hafnium or a combination of some of these elements.

23. Process according to one of the preceding claims, **characterized in that** the catalyst comprises manganese and cobalt.

24. Process according to one of the preceding claims, **characterized in that** the concentration of catalyst, expressed as manganese, in the oxidation medium is greater than 10 ppm.

25. Process according to one of the preceding claims, **characterized in that** the concentration of catalyst, expressed as manganese, in the oxidation medium is between 10 ppm and 5 000 ppm.

## Patentansprüche

1. Verfahren zur Oxidation von gegebenenfalls substituierten gesättigten aliphatischen oder cycloaliphatischen Kohlenwasserstoffen oder alkylaromatischen Kohlenwasserstoffen und/oder Alkoholen und Ketonen zu Säuren oder Polysäuren in flüssigem Medium mit einem molekularen Sauerstoff umfassenden Oxidationsmittel, **dadurch gekennzeichnet, daß** man die Reaktion in Gegenwart eines Katalysators auf Basis von Mangan und einer organischen Säureverbindung der folgenden allgemeinen Formel (I) duchführt: 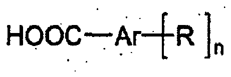 worin:
- Ar für einen aromatischen Rest mit einem aromatischen Ring oder mehreren aromatischen Ringen in kondensierter Form steht,
- n für eine ganze Zahl mit einem Wert von 1, 2 oder 3 steht,
- R für einen Rest der folgenden allgemeinen Formel (II) steht: 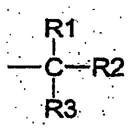
worin:
R1, R2 und R3 gleich oder verschieden sind und für eine Alkylkette mit 1 bis 4 Kohlenstoffatomen oder ein Fluor-, Chlor- oder Bromatom stehen.

2. Verfahren nach Anspruch 1, **dadurch gekennzeichnet, daß** der zu oxidierende Kohlenwasserstoff unter den Bedingungen der Durchführung der Oxidationsreaktion zumindest teilweise mit der Säureverbindung mischbar ist.

3. Verfahren nach einem der Ansprüche 1 bis 2, **dadurch gekennzeichnet, daß** man die Säureverbindung aus der Gruppe bestehend aus Benzoesäuren und Naphthalinsäuren auswählt.

4. Verfahren nach Anspruch 3, **dadurch gekennzeichnet, daß** die Benzoesäuren und Naphthalinsäuren durch tert.-Alkyl- oder Fluorkohlenwasserstoffgruppen substituiert sind.

5. Verfahren nach Anspruch 3 oder 4, **dadurch gekennzeichnet, daß** man die Säureverbindung aus der Gruppe bestehend aus 3,5-Di-tert.-butylbenzoesäure, 3,5-Ditrifluormethylbenzoesäure, 4-Trifluormethylbenzoesäure und 4-tert.-Butylbenzoesäure auswählt.

6. Verfahren nach einem der vorhergehenden Ansprüche, **dadurch gekennzeichnet, daß** der Gewichtsprozentanteil der Säureverbindung in dem flüssigen Medium zwischen 1 und 99 Gew.-%, bezogen auf das Gesamtgewicht des flüssigen Mediums, liegt.

7. Verfahren nach Anspruch 6, **dadurch gekennzeichnet, daß** der obige Gewichtsprozentanteil zwischen 2 und 50 Gew.-% liegt.

8. Verfahren nach einem der vorhergehenden Ansprüche, **dadurch gekennzeichnet, daß** der Katalysator unter den Bedingungen der Durchführung der Oxidationsreaktion in dem flüssigen Medium löslich ist.

9. Verfahren nach einem der Ansprüche 1 bis 7, **dadurch gekennzeichnet, daß** der Katalysator unter den Bedingungen der Durchführung der Oxidationsreaktion in dem flüssigen Medium unlöslich ist.

10. Verfahren nach Anspruch 9, **dadurch gekennzeichnet, daß** es sich bei dem Katalysator um einen geträgerten Katalysator, der einen anorganischen oder polymeren Träger umfaßt, handelt.

11. Verfahren nach einem der vorhergehenden Ansprüche, **dadurch gekennzeichnet, daß** man die Oxidation in Gegenwart einer Verbindung aus der Familie, die Nitrile, Hydroxyimidverbindungen und halogenierte Verbindungen umfaßt, durchführt.

12. Verfahren nach Anspruch 11, **dadurch gekennzeichnet, daß** es sich bei der obigen Verbindung um eine Nitrilverbindung aus der Gruppe, die Acetonitril und Benzonitril umfaßt, handelt.

13. Verfahren nach Anspruch 11, **dadurch gekennzeichnet, daß** es sich bei der obigen Verbindung um eine Hydroxyimidverbindung aus der Gruppe, die N-Hydroxysuccinimid und N-Hydroxyphthalimid umfaßt, handelt.

14. Verfahren nach Anspruch 11, **dadurch gekennzeichnet, daß** es sich bei der obigen Verbindung um eine halogenierte Verbindung aus der Gruppe, die cyclische oder acyclische fluorierte oder perfluorierte aliphatische Kohlenwasserstoffe oder fluorierte aromatische Kohlenwasserstoffe wie Perfluortoluol, Perfluormethylcyclohexan, Perfluorhexan, Perfluorheptan, Perfluoroctan, Perfluornonan, Perfluordecalin, Perfluormethyldecalin, α,α,α-Trifluortoluol oder 1,3-Bis(trifluormethyl)benzol, perfluorierte oder fluorierte Ester wie Perfluoralkyloctanoate oder Perfluoralkylnanoate, fluorierte oder perfluorierte Ketone wie Perfluoraceton, fluorierte oder perfluorierte Alkohole wie Perfluorhexanol, Perfluoroctanol, Perfluornonanol, Perfluordecanol, Perfluor-t-butanol, Perfluorisopropanol oder 1,1,1,3,3,3-Hexafluor-2-propanol, fluorierte oder perfluorierte Nitrile wie Perfluoracetonitril, fluorierte oder perfluorierte Säuren wie Trifluormethylbenzoesäure, Pentafluorbenzoesäure, Perfluorhexansäure, Perfluorheptansäure, Perfluoroctansäure, Perfluornonansäure oder Perfluoradipinsäure, fluorierte oder perfluorierte Halogenide wie Perfluoriodoctan oder Perfluorbromoctan, fluorierte oder perfluorierte Amine wie Perfluortripropylamin, Perfluortributylamin oder Perfluortripentylamin umfaßt, handelt.

15. Verfahren nach einem der vorhergehenden Ansprüche, **dadurch gekennzeichnet, daß** man den zu oxidierenden Kohlenwasserstoff aus der Gruppe, die Cyclohexan und Cyclododecan umfaßt, auswählt.

16. Verfahren nach Anspruch 15, **dadurch gekennzeichnet, daß** es sich bei der produzierten Säure um Adipinsäure oder Dodecandisäure handelt.

17. Verfahren nach einem der vorhergehenden Ansprüche, **dadurch gekennzeichnet, daß** man das flüssige Medium nach der Oxidation durch Absetzenlassen in mindestens eine organische Phase, die durch den nicht oxidierten Kohlenwasserstoff und die Säureverbindung gebildet wird, wobei die organischen Phasen in eine neue Oxidation zurückgeführt werden, und mindestens eine wäßrige Phase, die die produzierte Säure enthält, trennt.

18. Verfahren nach Anspruch 17, **dadurch gekennzeichnet, daß** man die Säure durch Kristallisation aus der wäßrigen Phase extrahiert.

19. Verfahren nach einem der Ansprüche 9 und 17 oder 18, **dadurch gekennzeichnet, daß** man den Katalysator mit der bzw. den organischen Phasen zurückführt.

20. Verfahren nach einem der Ansprüche 10 und 17 oder 18, **dadurch gekennzeichnet, daß** man den Katalysator durch Absetzenlassen oder Fest-Flüssig-Trennung von dem flüssigen Medium abtrennt.

21. Verfahren nach einem der Ansprüche 17 oder 18, **dadurch gekennzeichnet, daß** man den in der wäßrigen Phase löslichen Katalysator durch Flüssig-Flüssig-Extrakion, Abtrennung an Harzen oder Elektrodialyse extrahiert.

22. Verfahren nach einem der vorhergehenden Ansprüche, **dadurch gekennzeichnet, daß** der Katalysator Dotierungselemente aus der Gruppe, die Cobalt, Kupfer, Cer, Vanadium, Chrom, Brom, Zirconium, Hafnium oder eine Kombination einiger dieser Elemente umfaßt, umfaßt.

23. Verfahren nach einem der vorhergehenden Ansprüche, **dadurch gekennzeichnet, daß** der Katalysator Mangan und Cobalt umfaßt.

24. Verfahren nach einem der vorhergehenden Ansprüche, **dadurch gekennzeichnet, daß** die Katalysatorkonzentration, ausgedrückt als Mangan, im Oxidationsmedium über 10 ppm liegt.

25. Verfahren nach einem der vorhergehenden Ansprüche, **dadurch gekennzeichnet, daß** die Katalysatorkonzentration, ausgedrückt als Mangan, im Oxidationsmedium zwischen 10 ppm und 5000 ppm liegt.
